# FASCICULE DE BREVET EUROPEEN

(11) **EP 1 919 473 B1**
(45) Date de publication et mention de la délivrance du brevet: **05.12.2012**
(21) Numéro de dépôt: 06794228.4
(22) Date de dépôt: 27.07.2006
(51) Int. Cl.: A61K 31/437, A61K 31/15, A61P 25/00, A61P 43/00

(54) **ASSOCIATION D'UN AGENT HYPNOTIQUE A DUREE D'ACTION LONGUE ET D'UN AGENT HYPNOTIQUE A DUREE D'ACTION COURTE ET SON APPLICATION THERAPEUTIQUE**
KOMBINATION AUS SCHLAFMITTEL MIT LANGZEITWIRKUNG UND SCHLAFMITTEL MIT KURZZEITWIRKUNG SOWIE DEREN THERAPEUTISCHE VERWENDUNG
COMBINATION OF A LONG-ACTING HYPNOTIC AGENT AND A SHORT-ACTING HYPNOTIC AGENT AND THERAPEUTIC USE OF SAME

(30) Priorité: 19.08.2005 FR 0508643
(43) Date de publication de la demande: 14.05.2008
(73) Titulaire: SANOFI, 75008 Paris (FR)
(72) Inventeur: CUINE, Alain, F-77310 Saint Fargeau-Ponthierry (FR); DECOBERT, Michel, F-91190 Gif Sur Yvette (FR); FRANCON, Dominique, F-78370 Plaisir (FR); SAUNAL, Henry, F-34070 Montpellier (FR)
(74) Mandataire: Le Coupanec, Pascale A.M.P.
(86) Numéro de dépôt international: PCT/FR2006/001830
(87) Numéro de publication internationale: WO 2007/020337

(56) Documents cités:
- WO-A-2005/063297
- US-B1- 6 348 485
- DATABASE EMBASE [Online] ELSEVIER SCIENCE PUBLISHERS, AMSTERDAM, NL; 2002, TANAKA H ET AL: "Combined use of melatonin and low-dose flunitrazepam for treatment of sleep disturbance in a child with spastic quadriplegia: Evaluation using polysomnography" XP002380540 Database accession no. EMB-2002413692 & NO TO HATTATSU 2002 JAPAN, vol. 34, no. 6, 2002, pages 528-532, ISSN: 0029-0831

## Description

L'invention se rapporte à une association d'au moins un agent hypnotique à durée d'action longue et d'au moins un agent hypnotique à durée d'action courte. L'invention se rapporte également à une composition la contenant et à son application en thérapeutique.

Un certain nombre d'agents hypnotiques a été développé depuis des années, présentant des modes et des durées d'action variés.

Une première classe d'agents hypnotiques est ceux présentant une durée d'action courte. On entend dans ce qui suit par agent hypnotique à durée d'action courte un composé qui agit principalement comme inducteur de sommeil, c'est-à-dire agissant sur le temps d'entrée en phase de sommeil.

Ainsi, le zolpidem est un tel agent hypnotique à durée d'action courte, agissant comme modulateur des récepteurs GABA-A. Le zolpidem appartient à la classe des imidazopyridines, et est administré de façon orale sous forme de comprimé à libération immédiate ou sous une forme galénique permettant une libération retardée.
Le zolpidem agit rapidement, est bien absorbé avec une biodisponibilité de 70 %. Le dosage moyen, entre 5 et 10 mg dans une formulation conventionnelle, induit une concentration plasmatique maximale qui est atteinte entre 0,5 et 3 heures, le temps de demi-vie est court, avec une valeur moyenne de 2,4 heures et une durée d'action allant jusqu'à 6 heures.
D'autres exemples d'agents hypnotiques à durée d'action courte sont le zaleplon, qui appartient à la classe des pyrazolopyrimidines, le zopiclone, le eszopiclone, qui appartiennent à la classe des cyclopyrrolones.

Il a été développé également des agents hypnotiques à durée d'action longue. On entend dans ce qui suit par agent hypnotique à durée d'action longue un composé qui agit principalement sur la qualité et/ou le maintien du sommeil, notamment les phases de sommeil profond.

Un tel agent hypnotique à durée d'action longue est l'éplivansérine. L'éplivansérine est un inhibiteur des récepteurs 5HT2A qui agit sans blocage de la dopamine. L'éplivansérine, ainsi que sa préparation, est notamment décrite dans le document EP-A-0 373 998.
L'éplivansérine est également bien absorbée avec une biodisponibilité de 80 %. Le dosage conventionnel, entre 1 et 10 mg, induit une concentration plasmatique maximale qui est atteinte entre 2 et 6 heures, le temps de demi-vie étant relativement long, avec une valeur moyenne de 50 heures.
D'autres agents hypnotiques à durée d'action longue décrits dans le présent texte sont par exemple le gaboxadol et la pregabaline, ainsi que leurs dérivés.

Les agents hypnotiques décrits ci-dessus permettent de traiter les troubles du sommeil, notamment l'insomnie. Toutefois, tandis que les agents hypnotiques à durée d'action courte agissent principalement sur l'entrée en phase de sommeil, les agents hypnotiques à durée d'action longue agissent plutôt sur la phase de sommeil profond. En outre, les agents hypnotiques peuvent, notamment lorsqu'ils sont administrés à des doses élevées, avoir une incidence négative sur les périodes d'éveil, en particulier celle qui suit la prise du médicament. Il est ainsi toujours souhaitable de pouvoir proposer une composition permettant d'induire ou de maintenir un sommeil réparateur, et ce à une dose faible. Le document US 6,348,485 décrit un procédé de traitement des troubles du sommeil chez un mammifère comprenant l'administration au mammifère en ayant besoin d'une combinaison :
- d'un premier composé, (S)-N-[2-(1,6,7,8-tetrahydro-2H-indeno[5,4-b]furan-8yl)ethyl]propionamide en une teneur insuffisante pour entraîner le sommeil lorsqu'il est administré seul, et
- d'un second composé, à savoir au moins un composé actif choisi parmi le groupe constitué du zolpidem, du zopiclone, du brotizolam et du triazolam, en une teneur insuffisante pour entraîner le sommeil lorsqu'il est administré seul,
de manière à ce que cette combinaison entraîne le sommeil.

Le document WO 2005/063297 décrit un procédé de traitement d'un patient souffrant de troubles du sommeil ou d'insomnies comprenant l'administration d'une quantité efficace d'un point de vu thérapeutique d'une composition pharmaceutique comprenant un sédatif et de la mélatonine ou un analogue, le sédatif étant préférentiellement le (S)-zopiclone.

Le document EMB-2002 413692 (Tanaka *et al*., 2002) décrit quant à lui l'utilisation combinée de melatonine et d'une faible dose de flunitrazepam dans le traitement de désordre du sommeil chez un enfant manifestant une quadriplégie spastique.

L'invention a pour but de remédier à cet inconvénient, en proposant une association qui permet de combiner les actions des agents hypnotiques à durée d'action courte et longue, en améliorant encore la qualité du sommeil et les effets respectifs des agents hypnotiques à durée d'action courte et longue, sans effet négatif sur les phases d'éveil du patient.

Un premier but de l'invention concerne donc une association de deux agents hypnotiques.
Un autre objet de l'invention concerne une composition pharmaceutique renfermant une association de deux agents hypnotiques.
Un autre objet de l'invention concerne l'utilisation de cette association pour la préparation d'un médicament.

Selon un premier aspect, l'invention se rapporte à une association de deux agents hypnotiques.
L'association de l'invention comprend au moins un agent hypnotique à durée d'action courte et au moins un agent hypnotique à durée d'action longue.
Selon une forme de réalisation, l'agent hypnotique à durée d'action courte est présent sous une formulation galénique adaptée pour une libération immédiate ou prolongée, et l'agent hypnotique à durée d'action longue est présent sous une formulation galénique adaptée pour une libération immédiate.
Il a été en effet découvert que l'association d'un agent hypnotique à durée d'action courte avec un agent hypnotique à durée d'action longue permet d'obtenir un effet bénéfique sur le sommeil du patient, et que cet effet est supérieur à celui de chacun des deux agents hypnotiques considérés individuellement.

Selon un premier aspect de l'invention, l'agent hypnotique à durée d'action courte et l'agent hypnotique à durée d'action longue sont libérés de façon immédiate. Les deux agents apparaissent alors dans le plasma selon leurs caractéristiques pharmacocinétiques respectives. Ainsi l'agent hypnotique à durée d'action courte apparaît dans le plasma avant l'agent hypnotique à durée d'action longue.
Selon cette forme de réalisation, chaque agent développe son mécanisme d'action, avec un effet synergique entre les deux agents.

Selon un second aspect de l'invention, l'agent hypnotique à durée d'action courte est libéré de façon prolongée et l'agent hypnotique à durée d'action longue est libéré de façon immédiate. Selon cette forme de réalisation, le temps d'action de l'agent hypnotique à durée d'action courte est augmenté, avec un temps de résidence dans le plasma augmenté. Ainsi les deux agents peuvent agir en même temps, également avec un effet synergique.

On décrit dans le présent texte des exemples d'agents hypnotiques à durée d'action courte tels que notamment les modulateurs des récepteurs GABA-A, les benzodiazépines, les phénothiazines, les dérivés de mélatonine, les agonistes des récepteurs à la mélatonine.
L'agent hypnotique à durée d'action courte selon l'invention est choisi parmi le zolpidem, le zopiclone, le eszopiclone, le zaleplon, la melatonine, le ramelteon, le triazolam, l'etizolam, le brotizolam, l'indiplon ainsi que leurs dérivés et/ou mélanges.

On décrit dans le présent texte des exemples d'agents hypnotiques à durée d'action longue tels que notamment les antagonistes des récepteurs 5HT2A, les modulateurs des récepteurs GABA-A, les benzodiazépines, les modulateurs des ions calcium.
On décrit ainsi dans le présent texte les agents hypnotiques à durée d'action longue tels que le temazepam, le clonazepam, le gaboxadol, la pregabaline, ainsi que leurs dérivés et/ou mélanges.
Un agent hypnotique à durée d'action longue conforme à l'invention est choisi parmi l'éplivansérine, sous forme de base ou de sel d'addition, hydrate ou solvat, ainsi que leurs mélanges.

Les agents hypnotiques à durée d'action courte ou longue décrits ci-dessus peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.
Les agents hypnotiques à durée d'action courte ou longue décrits ci-dessus peuvent également exister à l'état de bases ou de sels d'addition à des acides. De tels sels d'addition font partie de l'invention.
Ces sels peuvent être préparés avec des acides pharmaceutiquement acceptables.

Les agents hypnotiques à durée d'action courte ou longue décrits ci-dessus peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Selon une forme d'exécution de l'invention, l'association comprend du zolpidem, notamment sous forme d'hémitartrate, en tant qu'agent hypnotique durée d'action courte et de l'éplivansérine, notamment sous forme de fumarate, en tant qu'agent hypnotique à durée d'action longue.

Selon un autre aspect, l'invention concerne des compositions pharmaceutiques comprenant, en tant que principe actif, au moins un agent hypnotique à durée d'action courte et au moins un agent hypnotique à durée d'action longue. Les compositions pharmaceutiques de l'invention contiennent une dose efficace d'au moins un agent hypnotique à durée d'action courte et d'au moins un agent hypnotique à durée d'action longue, ou un sel pharmaceutiquement acceptable de ces agents, un hydrate ou solvat desdits agents, ainsi qu'au moins un excipient pharmaceutiquement acceptable.
Les excipients sont choisis selon la forme pharmaceutique et le mode d'administration souhaité, parmi les excipients habituels qui sont connus de l'Homme du métier.
L'agent hypnotique à durée d'action courte et l'agent hypnotique à durée d'action longue peuvent être choisis parmi ceux décrits dans ce qui précède.

La composition pharmaceutique de l'invention est adaptée pour le traitement et la prévention des troubles du sommeil.
Dans le cadre de la présente demande de brevet, on entend par troubles du sommeil notamment les dyssomnies, les hypersomnies, les parasomnies, l'apnée du sommeil, l'insomnie, l'insomnie primaire, l'insomnie de maintenance du sommeil, l'insomnie liée à une maladie mentale, l'insomnie induite par une drogue telle que la caféine, l'alcool, les amphétamines, les opioïdes, les anxiolytiques.

Les formes unitaires d'administration appropriées comprennent les formes par voie orale telles que les comprimés, notamment les comprimés multicouche, enrobés, à noyau, les gélules molles ou dures, les poudres, les granules et les solutions ou suspensions orales, les formes d'administration sublinguale, buccale.

Selon une forme d'exécution, l'agent hypnotique à durée longue et l'agent hypnotique à durée d'action courte présents dans la composition selon l'invention sont libérés de façon immédiate.

Selon une autre forme d'exécution, l'agent hypnotique à durée d'action longue présent dans la composition selon l'invention est libéré de façon immédiate et l'agent hypnotique à durée d'action courte est libéré de façon prolongée.

L'entité à libération immédiate peut consister en une unité à libération immédiate d'un produit pharmaceutique telle que par exemple un comprimé ou une gélule à libération immédiate, ou plusieurs de ces unités sous forme de comprimé formulé dans une gélule; la matrice à libération immédiate d'un comprimé; une couche à libération immédiate incorporée dans un comprimé multicouche; une ou plusieurs couches d'enrobage dans un comprimé ou pellet.

L'entité à libération prolongée peut consister en une unité à libération prolongée d'un produit pharmaceutique telle que par exemple un comprimé ou gélule à libération prolongée; ou plusieurs de ces unités formulées dans une gélule; une couche à libération prolongée incorporée dans un comprimé multicouche; un noyau à libération prolongée ou une couche d'enrobage incorporée dans un comprimé à plusieurs enrobages; des pellets à libération prolongée à l'intérieur d'un comprimé se désagrégeant.

L'agent hypnotique à durée d'action longue et l'agent hypnotique à durée d'action courte peuvent être formulés selon l'invention dans une composition pharmaceutique unique ou, alternativement, dans des compositions pharmaceutiques séparées pour une administration simultanée, séparée ou séquentielle.

Par voie orale, la dose de principe actif présent dans une composition selon l'invention varie de 0,1 à 30 mg d'agent hypnotique à durée d'action longue et de 0,1 à 30 mg d'agent hypnotique à durée d'action courte.
Par exemple, une composition selon l'invention contient de 0,2 à 15 mg, notamment de 1 à 10 mg d'éplivansérine sous forme de base, et de 0,2 à 20 mg, notamment de 1 à 10 mg de zolpidem sous forme de base.
Il peut exister des cas particuliers où des dosages plus élevés ou plus faibles sont appropriés; de tels dosages ne sortent pas du cadre de l'invention. Selon la pratique habituelle, le dosage approprié à chaque patient est déterminé par le médecin selon le mode d'administration, le poids et la réponse dudit patient.

Un premier mode de réalisation des compositions selon l'invention consiste en une gélule comprenant un ou plusieurs comprimés à libération immédiate contenant l'agent hypnotique à durée d'action courte et un ou plusieurs comprimés à libération immédiate contenant l'agent hypnotique à durée d'action longue.

Un autre mode de réalisation des compositions selon l'invention consiste en une gélule comprenant un ou plusieurs comprimés à libération prolongée contenant l'agent hypnotique à durée d'action courte et un ou plusieurs comprimés à libération immédiate contenant l'agent hypnotique à durée d'action longue.

Un autre mode de réalisation des compositions selon l'invention consiste en une gélule comprenant un mélange de pellets à libération immédiate de l'agent hypnotique à durée d'action courte et de pellets à libération immédiate de l'agent hypnotique à durée d'action longue.

Un autre mode de réalisation des compositions selon l'invention consiste en une gélule comprenant un mélange de pellets à libération prolongée de l'agent hypnotique à durée d'action courte et de pellets à libération immédiate de l'agent hypnotique à durée d'action longue.

Un autre mode de réalisation des compositions selon l'invention consiste en un comprimé contenant des pellets à libération immédiate de l'agent hypnotique à durée d'action courte et de l'agent hypnotique à durée d'action longue.

Un autre mode de réalisation des compositions selon l'invention consiste en un comprimé contenant des pellets à libération prolongée de l'agent hypnotique à durée d'action courte et des pellets à libération immédiate de l'agent hypnotique à durée d'action longue.

Un autre mode de réalisation des compositions selon l'invention consiste en un comprimé revêtu entérique à libération prolongée comprenant des pellets à libération immédiate de l'agent hypnotique à durée d'action longue et des pellets à libération immédiate de l'agent hypnotique à durée d'action courte.

Un autre mode de réalisation des compositions selon l'invention consiste en un comprimé enrobé sec comprenant un noyau interne à libération prolongée renfermant l'agent hypnotique à durée d'action courte et une couche d'enrobage à libération immédiate renfermant l'agent hypnotique à durée d'action longue.

Les compositions selon l'invention peuvent être préparées selon les méthodes connues de l'homme du métier.

Ainsi, les gélules comprenant un ou plusieurs comprimés de taille réduite à libération immédiate contenant l'agent hypnotique à durée d'action longue et un ou plusieurs comprimés de taille réduite à libération immédiate contenant l'agent hypnotique à durée d'action courte peuvent être préparées de la façon suivante.

Les comprimés à libération immédiate peuvent être préparés par compression directe de mélanges des principes actifs sous forme de base ou de sels avec des diluants, tels que la cellulose microcristalline, le mannitol, le sorbitol, le lactose. D'autres excipients, tels que des désagrégeants ou des lubrifiants, peuvent être ajoutés.
Le choix entre ces excipients fonctionnels ainsi que ces diluants est bien connu de l'homme du métier.

Selon une autre forme d'exécution, les comprimés peuvent être préparés par granulation à l'eau ou aux solvants d'un mélange du ou des principes actifs avec les diluants, agents désagrégeants et polymère de liaison appropriés, puis calibration et séchage du granulat obtenu, addition d'un agent lubrifiant, suivie par une compression sur une machine à comprimer.
Les méthodes mises en oeuvre sont généralement celles décrites dans la littérature, par exemple B. B. Sheth, F. J. Bandelin, R. JF. Shangraw, Compressed tablets, dans Pharmaceutical dosage forms : Tablets, Vol 1, édité par H. A. Lieberman and L Lachman, Dekker N, Y. (1980).

Les gélules comprenant un ou plusieurs comprimés de taille réduite à libération immédiate contenant l'agent hypnotique à durée d'action longue et un ou plusieurs comprimés de taille réduite à libération prolongée contenant l'agent hypnotique à durée d'action courte peuvent être préparées de la façon suivante.

Des comprimés à libération prolongée contenant l'agent hypnotique à durée d'action courte peuvent être préparés par enrobage de comprimés à libération immédiate tel que décrits ci-dessus, avec un enrobage de polymère à diffusion limitée.
Des polymères à cette fin peuvent être choisis parmi les copolymères d'éthylcellulose ainsi que les polymères de méthacrylate de méthyle, tels que les produits commercialisés sous les dénominations Eudragit TM RS^{®}, Eudragit TM RL^{®}, Eudragit TM NE^{®}.

Les méthodes d'enrobage peuvent consister en la pulvérisation d'une solution du polymère sur les comprimés, dans un appareil à enrober ou un dispositif à lit fluidisé. Le solvant peut être organique ou aqueux, selon la nature du polymère utilisé. Des méthodes d'enrobage sont décrites notamment dans J. M. Bakan, Microencapsulation, dans L. Lachman, H. Lieberman et J. L. Kanig (Eds), The Theory and Practice of Industrial Pharmacy, Lea & Febinger, Philadelphia, USA, 1986 ; J. M. Mc Ginity, Aqueous Polymer Coatings for Pharmaceutical Dosage Forms, Dekker NY, 1989.

Les comprimés à libération prolongée peuvent également être préparés en incorporant des excipients formant la matrice dans la formulation, sans agent désagrégeant. Des exemples d'excipients formant matrice sont les polymères hydrophiles, notamment l'hydroxypropylméthylcellulose, l'hydroxyméthylcellulose, l'hydroxyéthylcellulose qui gonflent lorsqu'ils sont en contact avec des liquides aqueux, et qui peuvent contrôler la libération du principe actif à travers le réseau polymérique gonflé. De tels excipients sont utilisés en une quantité exprimée en pourcentage en poids de 10 % à 30 % par rapport au poids total du comprimé.
L'excipient formant matrice peut également être une substance lipidique, telle que de l'huile de castor hydrogénée de la cire de carnauba, utilisée en une quantité exprimée en pourcentage en poids de 10 % à 40 % par rapport au poids total du comprimé.

Les comprimés à libération prolongée peuvent être formulés, dans le cas de principes actifs basiques, avec un acide organique pharmaceutiquement acceptable, choisi parmi ceux indiqués ci-après, de façon à maintenir le pH du comprimé pendant sa dissolution dans les conditions de pH neutre de l'intestin grêle.
Des exemples d'acides organiques utilisables comprennent les acides maléique, tartrique, malique, fumarique, lactique, citrique, adipique, succinique et leurs sels acides lorsqu'ils existent, sous forme de racémates ou d'isomères.

Les gélules comprenant un mélange de pellets à libération immédiate des agents hypnotiques à durée d'action longue et courte peuvent être préparées de la façon suivante.
Les pellets à libération immédiate des agents hypnotique à durée d'action longue et courte peuvent être préparés par dépôt du principe actif en suspension dans l'eau avec par exemple de l'hydroxypropylméthylcellulose ou dans un solvant organique tel que l'éthanol, la povidone ou un autre polymère approprié agissant comme liant, sur un granule sphérique.
Un dispositif d'enrobage par lit fluidisé est généralement utilisé.
Les particules peuvent être agglomérées pour former des granules ou pellets sphériques, dans un granulateur-mélangeur à haute vitesse, ou un agglomérateur rotatif à lit fluidisé.
De telles méthodes sont décrites dans K. W. Olson, A. M. Mehta, Int. J. Phar. Tech & Prod. Mfr. 6 18-24, 1985. Les pellets peuvent également être préparés par extrusion de masses ou fondus humides suivie d'une sphéronisation, comme décrit par exemple dans C. Vervaet, L. Baert & J. P. Remon, Int. J. Pharm. 116 (1995) 131-146.

Les excipients utilisés sont typiquement ceux présentant de bonnes qualités plastiques tels que la cellulose microcristalline, le mannitol. De petites quantités d'un liant polymérique sont généralement ajoutées. Des agents surfactants tels que le dodécyl sulfate de sodium peuvent également être incorporés pour faciliter l'extrusion.

Les gélules comprenant un mélange de pellets à libération immédiate de l'agent hypnotique à durée d'action longue et de pellets à libération prolongée de l'agent hypnotique à durée d'action courte peuvent être préparées de la façon suivante.
Les pellets à libération immédiate peuvent être préparés comme décrit dans ce qui précède.
Les pellets à libération prolongée peuvent, dans le cas de principes actifs basiques, contenir un acide organique pharmaceutiquement acceptable ou un sel acide d'un tel acide organique, pour maintenir le pH local à l'intérieur du pellet pendant sa dissolution dans les conditions de neutralité de l'intestin grêle.

Alternativement, les pellets peuvent être revêtus d'une membrane sensible au pH, contenant un polymère soluble à un pH neutre et imperméable à un pH acide, tel que par exemple le produit Eudragit TM S^{®}, qui permet une perméation améliorée du principe actif à des pH de 5 et au-dessus, pour compenser la solubilité réduite du principe dans ces zones de pH.

Les comprimés comprenant plusieurs pellets à libération immédiate de l'agent hypnotique à durée d'action longue et de l'agent hypnotique à durée d'action courte peuvent être préparés de la façon suivante.
Les différents pellets peuvent être noyés dans une matrice ou la matrice elle-même peut contenir l'un des agents hypnotiques.
Les comprimés se désagrègent alors lorsqu'ils sont en contact avec un fluide, libérant le principe actif rapidement, ou les pellets à libération immédiate, ou à partir de l'enrobage des pellets à libération immédiate.

Les comprimés comprenant un ou plusieurs pellets à libération immédiate de l'agent hypnotique à durée d'action longue et un ou plusieurs pellets à libération prolongée de l'agent hypnotique à durée d'action courte peuvent être préparés de la façon suivante.
1) Le comprimé peut consister en un mélange de pellets à libération immédiate et de pellets à libération prolongée comprenant les principes actifs, noyés dans une matrice ne contenant pas de principe actif.
2) Alternativement, Les pellets contenant les deux agents hypnotiques peuvent être noyés dans une matrice contenant elle-même l'un des deux agents thérapeutiques.

Selon une autre forme, les pellets à libération prolongée peuvent être enrobés d'une couche comprenant le principe actif et des excipients, permettant une libération immédiate à partir de cette couche d'enrobage, noyée dans une matrice exempte de principe actif.
La matrice entourant les pellets est formulée de façon que la compression en comprimés n'interfère pas avec l'intégrité de la membrane entourant les pellets.
Le comprimé se désagrège lorsqu'il est en contact avec un fluide, en libérant l'agent à durée d'action longue rapidement, à partir de la matrice ou des pellets à libération immédiate, ou à partir des enrobages des pellets à libération immédiate, et en libérant ensuite l'agent à durée d'action courte, à partir des pellets à libération prolongée.

La composition pharmaceutique de l'invention peut également se présenter sous la forme d'un comprimé multicouche.

Un tel comprimé multicouche comprend :
- une ou plusieurs couches à libération immédiate, chacune contenant une dose d'agent hypnotique à durée d'action longue et éventuellement une dose d'agent hypnotique à durée d'action courte;
- une ou plusieurs couches à libération prolongée, chacune contenant une dose d'agent hypnotique à durée d'action courte et ;
- éventuellement une couche supplémentaire ne renfermant pas de principe actif mais comprenant des polymères hydrophiles tels que les dérivés de cellulose, par exemple de l'hydroxypropylcellulose, de l'hydroxyéthylcellulose, de l'hydroxyméthylcellulose, ou des agents diluants solubles tels que le lactose, le sorbitol, le mannitol, un ou plusieurs autres polymères hydrophiles et/ou un ou plusieurs autres excipients solubles, cette couche modulant la libération du principe actif à partir de la couche à libération prolongée.
Chaque couche contient éventuellement d'autres excipients, pour permettre une bonne compression, lubrification, liaison du comprimé.

Une autre forme de réalisation consiste en un noyau comprenant l'agent hypnotique à durée d'action courte, éventuellement avec un acide organique pharmaceutiquement acceptable. Le noyau est enrobé par une couche de polymère renfermant l'agent hypnotique à durée d'action longue qui est libéré rapidement ou immédiatement par contact avec les fluides, tandis que l'agent hypnotique à durée d'action courte est libérée à partir du noyau.
Eventuellement, le noyau et la couche d'enrobage peuvent être formulés pour permettre une libération dans le colon.
Chaque constituant du comprimé à enrobage multiple peut comprendre d'autres excipients, pour permettre une bonne compression, lubrification et liaison. Des procédés de préparation de comprimés multicouche et de comprimés à enrobage multiple sont décrits notamment dans W. C. Gunsel, Compression coated and layer tablets in pharmaceutical dosage forms : tablets, Vol 1, édité par H. A. Lieberman et L. Lachman, Dekker N. Y. (1980).

### Exemple 1. Etude des effets de l'association d'un modulateur des récepteurs GABA et d'un inhibiteur des récepteurs 5HT2A sur le sommeil.

Pour cette étude on utilise quatre groupes de rats mâles Sprague-Dawley, chaque groupe comprenant de 5 à 9 rats.

Le groupe A reçoit de l'éplivansérine (voie orale, hémifumarate) à une dose de 3 mg/kg p.o.

Le groupe B reçoit du zolpidem (voie orale, hémitartrate) à une dose de 3 mg/kg p.o.

Le groupe C reçoit (voie orale) en association 3 mg/kg p.o. d'hémifumarate d'éplivansérine et 3 mg/kg d'hémitartrate de zolpidem, les deux composés étant administrés à 5 minutes d'intervalle.

Enfin, le groupe D reçoit du zolpidem (voie orale, hémitartrate) à une dose de 10 mg/kg p.o.

Les données sont enregistrées au jour 0 (jour témoin), où les animaux reçoivent uniquement un véhicule (eau distillée et méthylcellulose) et au jour 1 où les animaux reçoivent les principes actifs.

Les données sont enregistrées pendant 6 heures chaque jour, les principes actifs étant administrés 15 minutes avant le début de l'enregistrement.

Les résultats obtenus sont donnés dans le tableau I ci-après.

**Tableau I**

| | **Groupe A** | **Groupe B** | **Groupe C** | **Groupe D** |
|---|---|---|---|---|
| Durée d'éveil | -3% | -2% | -18 %** | - 20 %** |
| Durée de sommeil NREM | 0 % | +4% | + 50 %** | + 33 %** |
| Latence d'apparition du sommeil NREM | + 0,6 min | - 6 min | - 1 min | - 1,2 min |
| Durée moyenne des périodes de sommeil NREM | + 32 %* | -15 % | + 58 %** | + 31 %** |
| Nombre moyen des périodes de sommeil NREM | - 23 %* | + 19 %* | -5 % | + 1 % |

| | | | | |
|---|---|---|---|---|
| *: p > 0,05 ** : p < 0,01 | | | | |

Dans le tableau I, les résultats sont exprimés en pourcentage par rapport aux groupes contrôles ne recevant que le véhicule, sauf indication contraire.

NREM : Non-Rapid Eye Movement.

Durée d'éveil : durée totale d'éveil pendant les 6 heures d'enregistrement.

Durée de sommeil NREM : durée totale de sommeil NREM pendant les 6 heures d'enregistrement.

Latence d'apparition du sommeil NREM : temps mesuré depuis le début de l'enregistrement jusqu'au moment de la première période de sommeil NREM.

Durée moyenne des périodes de sommeil NREM : durée du sommeil NREM / nombre de périodes de sommeil NREM pendant les 6 heures d'enregistrement.

Il apparaît du tableau I ci-dessus que la dose orale de 3 mg/kg d'éplivansérine n'a d'effet ni sur la durée d'éveil ni sur la durée de sommeil NREM, mais induit une augmentation de la durée moyenne des périodes de sommeil NREM (et une diminution du nombre moyen des périodes de sommeil NREM).
Le zolpidem à une dose de 3 mg/kg n'a pas non plus d'effet statistique significatif sur les variables du sommeil, excepté une augmentation du nombre moyen de périodes de sommeil NREM.

L'association éplivansérine à 3 mg/kg et zolpidem à 3 mg/kg induit une augmentation du temps de sommeil NREM liée à une forte augmentation de la durée moyenne des périodes de sommeil NREM, le nombre moyen de périodes de sommeil NREM restant pratiquement inchangé. L'effet hypnotique dure 3 heures environ chez les rats.

L'association de doses d'éplivansérine et de zolpidem inefficaces quand elles sont utilisées seules, permet donc d'obtenir un effet hypnotique marqué chez le rat, similaire à celui observé avec une dose plus forte de zolpidem utilisé seul.

De plus, le blocage des récepteurs 5HT2A par l'éplivansérine favorise le maintien des phases de sommeil NREM, comme cela est montré par l'augmentation de la durée moyenne des périodes de sommeil NREM.

L'association de l'invention permet donc d'obtenir un effet positif sur l'induction et la qualité du sommeil, effet qui n'est pas obtenu avec un seul agent hypnotique, même à une dose plus élevée.

### Exemple 2. Préparation d'une gélule contenant de l'éplivansérine et du zolpidem

On prépare ci-après une gélule contenant, sous forme de comprimés de faible taille, du fumarate d'éplivansérine en tant qu'agent hypnotique à durée d'action longue, à une dose de 1,18 mg et de l'hémitartartre de zolpidem en tant qu'agent hypnotique à durée d'action courte, à une dose de 6,22 mg.

Le comprimé d'éplivansérine contient les ingrédients indiqués dans le tableau Il ci-après.

**Tableau II**

| **Ingrédient** | **Pourcentage (%) (pds / pds)** |
|---|---|
| Éplivansérine micronisée | 2,36 |
| Lactose monohydraté¹ | 87,14 |
| Amidon gélatinisé² | 8 |
| Croscarmellose de sodium³ | 2 |
| Stéarate de magnésium | 0,5 |

| | |
|---|---|
| ¹ : Pharmatose DMV ² : Starch 1500 ³ : Ac-di-sol (FMC) | |

On prépare au préalable le mélange fumarate d'éplivansérine, lactose monohydraté, amidon gélatinisé, croscarmellose de sodium et stéarate de sodium. Le mélange est ensuite placé dans un mélangeur biconique pendant trente minutes. Le mélange homogène est ensuite comprimé en utilisant une machine à comprimer rotative classique sous la forme de comprimés de 50 mg.

Le comprimé d'hémitartrate de zolpidem a la composition indiquée dans le tableau III ci-après.

**Tableau III**

| **Ingrédient** | **Pourcentage (%) (pds / pds)** |
|---|---|
| Hémitartrate de zolpidem | 10,37 |
| Lactose | 83,73 |
| Cellulose microcristalline ⁴ | 10,0 |
| Hydroxypropylméthycellulose 606⁵ | 2,1 |
| Carboxyméthylcellulose de sodium | 3,2 |
| Stéarate de magnésium | 0,6 |

| | |
|---|---|
| ⁴ : Avicel (FMC) ⁵ : Pharmacoat 606 (Shin-Etsu) | |

On mélange ensemble l'hémitartrate de zolpidem, le lactose, la cellulose microcristalline, l'hydroxypropylméthylcellulose et la carboxyméthylcellulose de sodium, puis on granule à l'eau. Le granulat est ensuite séché et calibré. Le granulat est ensuite mélangé avec le stéarate de magnésium et comprimé en une masse de 60 mg par comprimé, en utilisant une machine à comprimer rotative. On introduit ensuite les comprimés dosés à 1,18 mg de fumarate d'éplivansérine et à 6,42 mg d'hémitartrate de zolpidem dans une gélule dure de gélatine. Les profils de dissolution des gélules peuvent être mesurés en utilisant un appareil II de la Pharmacopée US, avec deux milieux de dissolution :
- 900 ml d'acide chlorhydrique 0,01 M et
- 900 ml d'un tampon de phosphate de potassium 0,05 M à pH 6,8, maintenu à 37 +/-0,5 °C, avec agitation (50 t.p.min.)

### Exemple 3. Préparation d'une gélule comprenant un comprimé à libération immédiate d'éplivansérine et un comprimé à libération prolongée de zolpidem.

On prépare les comprimés à libération immédiate de fumarate d'éplivansérine selon le procédé décrit dans l'exemple 2 ci-dessus.

On prépare le comprimé à libération prolongée d'hémitartrate de zolpidem selon la méthode décrite dans l'exemple 2 ci-dessus pour obtenir un comprimé présentant la composition indiquée dans le tableau IV ci-après.

**Tableau IV**

| **Ingrédients** | **Pourcentage (%) (pds/pds)** |
|---|---|
| Hémitartrate de zolpidem | 12,4 |
| Lactose monohydraté⁶ | 33,4 |
| Hydroxypropylméthylcellulose 4000 mPa.s⁷ | 25,0 |
| Cellulose microcristailine⁸ | 20,0 |
| Tartrate de potassium hydrogène | 8,0 |
| Stéarate de magnésium | 1,0 |
| Silice anhydre colloïdale | 0,2 |
| Eau purifiée | q.s. |

| | |
|---|---|
| ⁶ : Pharmatose (DMV) ⁷ : Metolose 90SH4000 (Shin-Etsu) ⁸ : Avicel PH 102 (FMC) | |

On utilise les mêmes méthodes de granulation humide et de compression que celles décrites pour l'hémitartrate de zolpidem dans l'exemple 2 ci-dessus. On prépare des gélules contenant un ou des comprimés à libération prolongée de 50 mg contenant 5 mg de zolpidem base (correspondant à 6,22 mg d'hémitartrate de zolpidem) et un ou des comprimés à libération immédiate de 50 mg contenant 1 mg d'éplivansérine base (correspondant à 1,18 mg de fumarate d'éplivansérine).
Les profils de dissolution *in vitro* des gélules ainsi préparées peuvent être établis en utilisant la méthode décrite dans l'exemple 2 ci-dessus.

### Exemple 4. Préparation d'une gélule comprenant un mélange de pellets à libération immédiate d'éplivansérine et de pellets à libération immédiate de zolpidem.

On prépare une suspension de 59 g de fumarate d'éplivansérine (correspondant à 50 g d'éplivansérine base) et de 100 g de povidone (Pladone K29/32, BASF) dans 670 g d'éthanol. 750 g de cette suspension sont ensuite pulvérisés sur 1060 g de microgranules de taille 16-18 mesh, en utilisant un sécheur à lit fluidisé.
On prépare ensuite une suspension de 62,2 g de tartrate de zolpidem (correspondant à 50g de zolpidem base) et de 100 g de povidone (Pladone K29/32, BASF) dans 670 g d'éthanol. 750 g de cette suspension sont ensuite pulvérisés sur 1060 g de microgranules de taille 16-18 mesh, en utilisant un sécheur à lit d'air fluidisé.
On prépare un mélange des deux pellets, avec un ratio de 1 partie en poids de fumarate d'éplivansérine pour 5 parties de tartrate de zolpidem. Ce mélange est introduit dans une gélule dure de gélatine, pour donner une quantité totale de 1 mg d'éplivansérine sous forme de base (correspondant à 1,18 mg de fumarate d'éplivansérine) et 5 mg de zolpidem sous forme de base (correspondant à 6,22 mg de tartrate de zolpidem). La quantité de chacun des pellets peut être modifiée pour ajuster la dose.
Les profils de dissolution *in vitro* des gélules ainsi préparées peuvent être établis en utilisant la méthode décrite dans l'exemple 2 ci-dessus.

### Exemple 5. Préparation d'une gélule comprenant un mélange de pellets à libération immédiate d'éplivansérine et de pellets à libération prolongée de zolpidem.

Les pellets à libération immédiate de fumarate d'éplivansérine sont préparés tel que décrit dans l'exemple 4 ci-dessus.

Des pellets d'hémitartrate de zolpidem sont préparés tel que décrit dans l'exemple 4 ci-dessus.

On prépare une solution comprenant 25 g de copolymère méthacrylate (Eudragit TM RL 100, Rohm Pharma), 143 g de copolymère méthacrylate (Eudragit TM RS 100, Rohm Pharma) et 18,7 g de citrate d'éthyle (Eudrafex TM, Rohm Pharma) dans 1180 g d'un mélange isopropanol / acétone 60 : 40 (pds/pds).

Les pellets d'hémitartrate de zolpidem sont enrobés avec ce mélange de polymères, par pulvérisation dans un sécheur à lit fluidisé, la quantité finale d'enrobage représentant 20 % en poids de la masse de pellet non enrobé.

Après maturation des pellets à 35°C pendant 24 heures, on prépare un mélange des pellets enrobés d'hémitartrate de zolpidem et des pellets de fumarate d'éplivansérine, dans la proportion de 1 : 2 (éplivansérine / zolpidem), et ce mélange est introduit dans des gélules de gélatine pour donner une quantité par gélule correspondant à 5 mg d'éplivansérine base et de 10 mg de zolpidem base.

Les profils de dissolution *in vitro* des gélules ainsi préparées peuvent être établis en utilisant la méthode décrite dans l'exemple 2 ci-dessus.

### Exemple 6. Préparation d'un comprimé comprenant des pellets à libération immédiate d'éplivansérine et des pellets à libération immédiate de zolpidem.

Les pellets de fumarate d'éplivansérine et d'hémitartrate de zolpidem sont préparés selon la méthode décrite dans l'exemple 4 ci-dessus.

On prépare un mélange des deux pellets dans un ratio en poids de 1 partie de fumarate d'éplivansérine pour 2 parties d'hémitartrate de zolpidem, et on ajoute 0,1 % de stéarate de magnésium. Le mélange est ensuite placé dans un mélangeur biconique pendant 30 minutes.

Le mélange homogène est ensuite comprimé en utilisant une machine à comprimer rotative classique, pour donner un comprimé renfermant 5,9 mg de fumarate d'éplivansérine (correspondant à 5 mg d'éplivansérine sous forme de base) et 12,44 mg d'hémitartrate de zolpidem (correspondant à 10 mg de zolpidem sous forme de base). Les profils de dissolution *in vitro* des gélules ainsi préparées peuvent être établis en utilisant la méthode décrite dans l'exemple 2 ci-dessus.

### Exemple 7. Préparation d'un comprimé comprenant des pellets à libération immédiate d'éplivansérine et des pellets à libération prolongée de zolpidem.

On prépare les pellets à libération immédiate de fumarate d'éplivansérine selon le procédé décrit dans l'exemple 4 et des pellets à libération prolongée de zolpidem selon le procédé décrit dans l'exemple 5.
On prépare un mélange des deux pellets dans un ratio en poids de 2 parties de fumarate d'éplivansérine pour 6 parties d'hémitartrate de zolpidem, et on ajoute 0,2 % de fumarate stearyl de magnésium. On transfère ensuite le mélange dans un mélangeur biconique pendant 30 minutes. Le mélange homogénéisé est ensuite comprimé en utilisant une machine à comprimer rotative classique, pour obtenir des comprimés contenant une quantité totale de 4,72 mg de fumarate d'éplivansérine (correspondant à 4 mg d'éplivansérine base) et de 14,93 mg d'hémitartrate de zolpidem (correspondant à 12 mg de zolpidem base).
Les profils de dissolution *in vitro* des gélules ainsi préparées peuvent être établis en utilisant la méthode décrite dans l'exemple 2 ci-dessus.

### Exemple 8. Préparation d'un comprimé entérique enrobé à libération prolongée comprenant des pellets à libération immédiate d'éplivansérine et des pellets à libération immédiate de zolpidem.

On prépare des comprimés comprenant à la fois du fumarate d'éplivansérine et de l'hémitartrate de zolpidem selon le procédé décrit dans l'exemple 6.

On enrobe ensuite les comprimés selon le procédé décrit ci-après.

On prépare une solution de 46 g de copolymère méthacrylate (Eudragit TM RL100, Rohm Pharma), 295 g de copolymère méthacrylate (Eudragit TM RS100, Rohm Pharma) et 40 g de citrate d'éthyle (Eudrafex TM, Rohm Pharma) dans 2280 g d'un mélange isopropanol / acétone 65 : 35 (pds/pds).

Les comprimés comprenant 3,93 mg de fumarate d'éplivansérine et 12,44 mg d'hémitartrate de zolpidem sont enrobés avec le mélange polymérique, par pulvérisation dans un système de type "coating pan", la quantité finale d'enrobage étant de 5 à 10 % en poids de la masse de pellet sans enrobage.

### Exemple 9. Préparation d'un comprimé bicouches comprenant une couche à libération immédiate d'éplivansérine et une couche à libération immédiate de zolpidem.

On prépare des granulats A par mélange à sec et des granulats B par mélange humide tel que décrit dans l'exemple 2 et selon les compositions indiquées dans le tableau V ci-après.

**Tableau V**

| **Ingrédients** | **Pourcentage (%) (Poids/poids)** |
|---|---|
| **Granulats A** | |
| Fumarate d'éplivansérine | 2,95 |
| Lactose monohydraté sec⁹ | 82,71 |
| Amidon prégélatinisé¹⁰ | 8,00 |
| Croscarmellose¹¹ | 2,00 |
| Carboxyméthylcellulose de sodium¹² | 3,80 |
| Stéarate de magnésium¹³ | 0,54 |

| **Granulats B** | |
|---|---|
| Hémitartrate de zolpidem | 6,22 |
| Lactose monohydraté⁹ | 73,88 |
| Cellulose microcristalline¹⁴ | 14,0 |
| Hydroxypropylméthylcellulose 606¹⁵ | 2,1 |
| Carboxyméthylcellulose de sodium¹² | 3,2 |
| Stéarate de magnesium¹³ | 0,6 |

| | |
|---|---|
| ⁹ : Pharmatose (DMV) ¹⁰ : Starch 1500 (Colorcon) ¹¹ : Ac-di-sol (FMC) ¹² : Blanose (Aqualon) ¹³ : Brentag AG ¹⁴ : Avicel PH 102 (FMC) ¹⁵ : Pharmacoat 606 (Shin-Etsu) | |

On comprime ensuite les mélanges en un comprimé bicouches en utilisant une machine à comprimer alternative, la première couche à libération immédiate d'une masse de 200 mg du granulat A comprenant 5,90 mg de fumarate d'éplivansérine (correspondant à 5 mg d'éplivansérine base) et la seconde couche à libération immédiate d'une masse de 200 mg du granulat B comprenant 12,44 mg d'hémitartrate de zolpidem (correspondant à 10 mg de zolpidem base).
Les profils de dissolution *in vitro* des gélules ainsi préparées peuvent être établis en utilisant la méthode décrite dans l'exemple 2 ci-dessus.

### Exemple 10. Préparation d'un comprimé bicouches comprenant une couche à libération immédiate d'éplivansérine et une couche à libération prolongée de zolpidem.

On prépare des granulats C par mélange à sec et des granulats D par mélange humide tel que décrit dans l'exemple 2 et selon les compositions indiquées dans le tableau VI ci-après.

**Tableau VI**

| **Ingrédients** | **Pourcentage (%) (Poids/poids)** |
|---|---|
| **Granulats C** | |
| Fumarate d'éplivansérine | 2,95 |
| Lactose monohydraté sec¹⁶ | 84,00 |
| Amidon prégélatinisé¹⁷ | 7,70 |
| Croscarmellose | 2,00 |
| Carboxyméthylcellulose de sodium¹⁹ | 3,4 |
| Stéarate de magnésium²⁰ | 0,54 |

| **Granulats D** | |
|---|---|
| Hémitartrate de zolpidem | 7,75 |
| Lactose 150 mesh¹⁶ | 37,85 |
| Cellulose microcristalline²¹ | 20,0 |
| Acide tartrique (23) | 8,4 |
| Hydroxypropylméthylcellulose²² | 25,0 |
| Stéarate de magnésium²³ | 1,0 |

| | |
|---|---|
| ¹⁶ : Pharmatose (DMV) ¹⁷ : Starch 1500 (Colorcon) ¹⁸ : Ac-di-sol (FMC) ¹⁹ : Blanose (Aqualon) ²⁰ : Brentag AG ²¹ : Avicel PH 102 (FMC) ²² : Metolose 90SH4000 (Shin-Etsu) ²³ : Brentag AG | |

On comprime les mélanges en un comprimé bicouches en utilisant une machine à comprimer alternative, la première couche à libération immédiate d'une masse de 150 mg du granulat C comprenant 4,425 mg de fumarate d'éplivansérine (correspondant à 3,75 mg d'éplivansérine base) et la seconde couche à libération prolongée d'une masse de 200 mg du granulat D comprenant 15,50 mg d'hémitartrate de zolpidem (correspondant à 12,45 mg de zolpidem base).
Les profils de dissolution *in vitro* des gélules ainsi préparées peuvent être établis en utilisant la méthode décrite dans l'exemple 2 ci-dessus.

### Exemple 11. Préparation d'un comprimé à trois couches comprenant une couche à libération immédiate d'éplivansérine, une couche inactive et une troisième couche à libération prolongée de zolpidem.

On prépare des granulats E et F par mélange à sec et des granulats G par mélange humide tel que décrit dans l'exemple 2 et selon les compositions indiquées

**Tableau VII**

| **Ingrédients** | **Pourcentage (%) (Poids/poids)** |
|---|---|
| **Granulats E (libération immédiate)** | |
| Fumarate d'éplivansérine | 2,36 |
| Lactose monohydraté sec²⁴ | 87,14 |
| Amidon prégélatinisé²⁵ | 8,0 |
| Croscarmellose²⁶ | 2,0 |
| Carboxyméthylcellulose de sodium²⁷ | 3,8 |
| Stéarate de magnésium²⁸ | 0,54 |
| **Granulats F (inactive)** | |
| Lactose monohydraté sec²⁴ | 60,0 |
| Cellulose microcristalline²⁹ | 24,0 |
| Acide tartrique³⁰ | 10,0 |
| Hydroxyéthylcellulose | 5,0 |
| Stéarate de magnésium²⁸ | 1,0 |

| **Granulats G (libération prolongée)** | |
|---|---|
| Hémitartrate de zolpidem | 5,0 |
| Lactose 200 mesh²⁴ | 67,7 |
| Cellulose microcristalline²⁹ | 20,0 |
| Hydroxypropylméthylcellulose 606³¹ | 2,5 |
| Carboxyméthylcellulose de sodium²⁷ | 3,8 |
| Stéarate de magnesium²⁸ | 1,0 |

| | |
|---|---|
| ²⁴ : Pharmatose (DMV) ²⁵ : Starch 1500 (colorcon) ²⁶ : Ac-di-sol (FMC) ²⁷ : Blanose (Aqualon) ²⁸ : Brentag AG ²⁹ _{:} Avicel PH 102 (FMC) ³⁰ : Brentag AG ³¹ : Pharmacoat (Shin-Etsu) | |

On comprime les mélanges tel que décrit dans l'exemple 9 en comprimé à trois couches, une couche externe d'une masse de 125 mg du granulat E comprenant 2,95 mg de fumarate d'éplivansérine (correspondant à 2,5 mg d'éplivansérine base), une couche intermédiaire d'une masse de 125 mg du granulat F et une troisième couche externe d'une masse de 300 mg du granulat G comprenant 15 mg d'hémitartrate de zolpidem (correspondant à 12,06 mg de zolpidem base).

### Exemple 12. Préparation d'un comprimé enrobé sec comprenant un noyau interne de zolpidem et un enrobage externe d'éplivansérine.

On prépare des granulats de la façon décrite dans l'exemple 2, sur la base des compositions indiquées dans le tableau VIII ci-après.

**Tableau VIII**

| **Ingrédients** | **Pourcentage (%) (Poids/poids)** |
|---|---|
| **Noyau interne (libération prolongée)** | |
| Hémitartrate de zolpidem | 15,55 |
| Lactose monohydraté 200 mesh³² | 36,05 |
| Cellulose microcristalline³³ | 18,0 |
| Hydroxypropylméthylcellulose | 21,0 |
| Acide tartrique³⁵ | 8,4 |
| Stéarate de magnesium³⁵ | 1,0 |

| **Enrobage externe (libération immédiate)** | |
|---|---|
| Fumarate d'éplivansérine | 1,96 |
| Lactose monohydraté 150 mesh³² | 52,00 |
| Cellulose microcristalline³³ | 39,84 |
| Hydroxypropylméthylcellulose 606³⁴ | 2,2 |
| Carboxyméthylcellulose de sodium³⁶ | 3,0 |
| Stéarate de magnesium³⁵ | 1,0 |

| | |
|---|---|
| ³² : Pharmatose (DMV) ³³ : Avicel PH 102 (FMC) ³⁴ : Metolose 90SH4000 (Shin-Etsu) ³⁵ : Brentag AG ³⁶ : Blanose (Aqualon) | |

Le granulat formant le noyau interne est compressé en petits comprimés en utilisant une machine à compresser alternative, avant d'effectuer l'opération d'enrobage à sec avec la seconde couche. Cette opération donne des comprimés à libération prolongée de 80 mg, contenant 12,44 mg d'hémitartrate de zolpidem (correspondant à 10 mg de zolpidem base).
Le granulat formant la couche externe d'enrobage est comprimé en utilisant une machine à compresser rotative, permettant d'inclure les petits comprimés de noyau interne. La couche externe possède une masse de 301 mg et renferme 5,9 mg de fumarate d'éplivansérine (correspondant à 5 mg d'éplivansérine base).

Selon un autre de ses aspects, l'invention a pour objet l'utilisation d'au moins un agent hypnotique à durée d'action longue en combinaison avec au moins un agent hypnotique à durée d'action courte, pour la préparation d'un médicament destiné à prévenir et/ou à traiter les troubles du sommeil tels que décrits dans ce qui précède, notamment l'insomnie.

## Revendications

1. Association d'au moins un agent hypnotique à durée d'action courte choisi parmi le zolpidem, le zopiclone, le eszopiclone, le zaleplon, la mélatonine, le ramelteon, le triazolam, l'etizolam, le brotizolam, l'indiplon, sous forme de base ou de sel d'addition, hydrate ou solvat, ainsi que leurs mélanges avec au moins un agent hypnotique à durée d'action longue choisi parmi l'éplivansérine, sous forme de base ou de sel d'addition, hydrate ou solvat, ainsi que leurs mélanges.

2. Association selon la revendication 1, **caractérisée en ce que** l'agent hypnotique à durée d'action courte est présent sous une formulation galénique adaptée pour une libération immédiate ou prolongée, et l'agent hypnotique à durée d'action longue est présent sous une formulation galénique adaptée pour une libération immédiate.

3. Composition pharmaceutique, **caractérisée en ce qu'**elle comprend, en tant que principe actif, au moins un agent hypnotique à durée d'action courte choisi parmi le zolpidem, le zopiclone, le eszopiclone, le zaleplon, la mélatonine, le ramelteon, le triazolam, l'etizolam, le brotizolam, l'indiplon, sous forme de base ou de sel d'addition, hydrate ou solvat, ainsi que leurs mélanges et au moins un agent hypnotique à durée d'action longue choisi parmi l'éplivansérine, sous forme de base ou de sel d'addition, hydrate ou solvat, ainsi que leurs mélanges, ainsi qu'au moins un excipient pharmaceutiquement acceptable.

4. Composition selon la revendication 3, **caractérisée en ce que** l'agent hypnotique à durée d'action courte est le zolpidem ou l'un de ses sels et **en ce que** l'agent hypnotique à durée d'action longue est l'éplivansérine ou l'un de ses sels.

5. Composition selon l'une quelconque des revendications 3 ou 4, **caractérisée en ce que** l'agent hypnotique à durée d'action courte et l'agent hypnotique à durée d'action longue sont libérés de façon immédiate.

6. Composition selon l'une quelconque des revendications 3 à 5, **caractérisée en ce que** l'agent hypnotique à durée d'action courte est libéré de façon prolongée et **en ce que** l'agent hypnotique à durée d'action longue est libéré de façon immédiate.

7. Composition selon l'une quelconque des revendications 3 à 6, **caractérisée en ce qu'**elle consiste en une gélule comprenant un ou plusieurs comprimés à libération immédiate contenant l'agent hypnotique à durée d'action courte et un ou plusieurs comprimés à libération immédiate contenant l'agent hypnotique à durée d'action longue.

8. Composition selon l'une quelconque des revendications 3 à 6, **caractérisée en ce qu'**elle consiste en une gélule comprenant un ou plusieurs comprimés à libération prolongée contenant l'agent hypnotique à durée d'action courte et un ou plusieurs comprimés à libération immédiate contenant l'agent hypnotique à durée d'action longue.

9. Composition selon l'une quelconque des revendications 3 à 6, **caractérisée en ce qu'**elle consiste en une gélule comprenant un mélange de pellets à libération immédiate de l'agent hypnotique à durée d'action courte et de pellets à libération immédiate de l'agent hypnotique à durée d'action longue.

10. Composition selon l'une quelconque des revendications 3 à 6, **caractérisée en ce qu'**elle consiste en une gélule comprenant un mélange de pellets à libération prolongée de l'agent hypnotique à durée d'action courte et de pellets à libération immédiate de l'agent hypnotique à durée d'action longue.

11. Composition selon l'une quelconque des revendications 3 à 6, **caractérisée en ce qu'**elle consiste en un comprimé contenant des pellets à libération immédiate de l'agent hypnotique à durée d'action courte et de l'agent hypnotique à durée d'action longue.

12. Composition selon l'une quelconque des revendications 3 à 6, **caractérisée en ce qu'**elle consiste en un comprimé contenant des pellets à libération prolongée de l'agent hypnotique à durée d'action courte et des pellets à libération immédiate de l'agent hypnotique à durée d'action longue.

13. Composition selon l'une quelconque des revendications 3 à 6, **caractérisée en ce qu'**elle consiste en un comprimé revêtu entérique à libération prolongée comprenant des pellets à libération immédiate de l'agent hypnotique à durée d'action longue et des pellets à libération immédiate de l'agent hypnotique à durée d'action courte.

14. Composition selon l'une quelconque des revendications 3 à 6, **caractérisée en ce qu'**elle consiste en un comprimé multicouche comprenant :
(a) une ou plusieurs couches à libération immédiate, chacune contenant une dose d'agent hypnotique à durée d'action longue et éventuellement une dose d'agent hypnotique à durée d'action courte,
(b) une ou plusieurs couches à libération prolongée, chacune contenant une dose d'agent hypnotique à durée d'action courte, et éventuellement
(c) une couche inactive ne renfermant pas d'agent hypnotique.

15. Composition selon l'une quelconque des revendications 3 à 6, **caractérisée en ce qu'**elle consiste en un comprimé enrobé sec, **caractérisée en ce qu'**elle comprend un noyau interne à libération prolongée renfermant l'agent hypnotique à durée d'action courte et **en ce que** la couche d'enrobage à libération immédiate renferme l'agent hypnotique à durée d'action longue.

16. Utilisation d'au moins un agent hypnotique à durée d'action courte, choisi parmi le zolpidem, le zopiclone, le eszopiclone, le zaleplon, la mélatonine, le ramelteon, le
triazolam, l'etizolam, le brotizolam, l'indiplon, sous forme de base ou de sel d'addition, hydrate ou solvat, ainsi que leurs mélanges, en combinaison avec au moins un agent hypnotique à durée d'action longue, choisi parmi l'éplivansérine, sous forme de base ou de sel d'addition, hydrate ou solvat, -ainsi que leurs mélanges, pour la préparation d'un médicament destiné à prévenir et/ou à traiter les troubles du sommeil, notamment les dyssomnies, les hypersomnies, les parasomnies, l'apnée du sommeil, l'insomnie, l'insomnie primaire, l'insomnie de maintenance du sommeil, l'insomnie liée à une maladie mentale, l'insomnie induite par une drogue telle que la caféine, l'alcool, les amphétamines, les opioïdes, les anxiolytiques.

## Claims

1. Combination of at least one short-acting hypnotic agent chosen from zolpidem, zopiclone, eszopiclone, zaleplon, melatonin, ramelteon, triazolam, etizolam, brotizolam and indiplon, in the form of base or of addition salt, hydrate or solvate, and also mixtures thereof with at least one long-acting hypnotic agent chosen from eplivanserine, in the form of base or of addition salt, hydrate or solvate, and also mixtures thereof.

2. Combination according to Claim 1, **characterized in that** the short-actin hypnotic agent is present in a galenical formulation suitable for immediate or sustained release, and the long-acting hypnotic agent is present in a galenical formulation suitable for immediate release.

3. Pharmaceutical composition, **characterized in that** it comprises, as active principle, at least one short-acting hypnotic agent chosen from zolpidem, zopiclone, eszopiclone, zaleplon, melatonin, ramelteon, triazolam, etizolam, brotizolam and indiplon, in the form of base or of addition salt, hydrate or solvate, and also mixtures thereof, and at least one long-acting hypnotic agent chosen from eplivanserine, in the form of base or of addition salt, hydrate or solvate, and also mixtures thereof, and also at least one pharmaceutically acceptable excipient.

4. Composition according to Claim 3, **characterized in that** the short-acting hypnotic agent is zolpidem or a salt thereof and **in that** the long-acting hypnotic agent is eplivanserine or a salt thereof.

5. Composition according to either of Claims 3 and 4, **characterized in that** the short-acting hypnotic agent and the long-acting hypnotic agent are released immediately.

6. Composition according to any one of Claims 3 to 5, **characterized in that** the short-acting hypnotic agent is released in a sustained manner and **in that** the long-acting hypnotic agent is released immediately.

7. Composition according to any one of Claims 3 to 6, **characterized in that** it consists of a gel capsule comprising one or more immediate-release tablets containing the short-acting hypnotic agent and one or more immediate-release tablets containing the long-acting hypnotic agent.

8. Composition according to any one of Claims 3 to 6, **characterized in that** it consists of a gel capsule containing one or more sustained-release tablets containing the short-acting hypnotic agent and one or more immediate-release tablets containing the long-acting hypnotic agent.

9. Composition according to any one of Claims 3 to 6, **characterized in that** it consists of a gel capsule containing a mixture of immediate-release pellets of the short-acting hypnotic agent and of immediate-release pellets of the lone-acting hypnotic agent.

10. Composition according to any one of Claims 3 to 6, **characterized in that** it consists of a gel capsule comprising a fixture of sustained-release pellets of the short-acting hypnotic agent and of immediate-release pellets of the long-acting hypnotic agent,

11. Composition according to any one of Claims 3 to 6, **characterized in that** it consists of a tablet containing immediate-release pellets of the short-acting hypnotic agent and of the lone-acting hypnotic agent.

12. Composition according to any one of Claims 3 to 6, **characterized in that** it consists of a tablet containing sustained-release pellets of the short-acting hypnotic agent and immediate-release pellets of the long-acting hypnotic agent.

13. Composition according to any one of Claims 3 to 6, **characterized in that** it consists of a sustained-release enteric-coated tablet comprising immediate-release pellets of the lone-acting hypnotic agent and immediate-release pellets of the short-acting hypnotic agent.

14. Composition according to any one of Claims 3 to 6, **characterized in that** it consists of a multilayer tablet comprising:
(a) one or more immediate-release layers, each containing a dose of long-acting hypnotic agent and optionally a dose of short-acting hypnotic agent,
(b) one or more sustained-release layers, each containing a dose of short-acting hypnotic agent, and optionally
(c) an inactive layer not containing any hypnotic agent.

15. Composition according to any one of Claims 3 to 6, **characterized in that** it consists of a dry-coated tablet, **characterized in that** it comprises an inner sustained-release core containing the short-acting hypnotic agent and **in that** the immediate-release coasting layer contains the long-acting hypnotic agent.

16. Use of at least one short-acting hypnotic agent chosen from zolpidem, zopiclone, eszopiclone, zaleplon, melatonin, ramelteon, triazolam, etizolam, brotizolam and indiplon, in the form of base or of addition salt, hydrate or solvate, and also mixtures thereof, in combination with at least one long-acting hypnotic-agent chosen from eplivanserine, in the form of base or of addition salt, hydrate or solvate, and also mixtures thereof, for the preparation of a medicament for presenting and/or treading sleep disorders, especially dyssomnia, hypersomnia, parasomnia, sleep apnea, insomnia, primary insomnia, sleep maintenance insomnia, insomnia associated with a mental disease, and insomnia induced by a drug such as caffeine, alcohol, amphetamines, opioids or anxiolytics.

## Patentansprüche

1. Vereinigung von mindestens einem Hypnotikum mit kurzer Wirkungsdauer, das ausgewählt wind aus Zolpidem, Zopiclon, Eszopiclon, Zaleplon, Melatonin, Ramelteon, Triazolam, Etizolam, Brotizolam, Indiplon, in Form der Base oder eines Additionssalzes, Hydrats oder Solvats sowie deren Gemische, mit mindestens einem Hypnotikum mit langer Wirkungsdauer, das ausgewählt wird aus Eplivanserin, in Form der Base oder eines Additionssalzes, Hydrats oder Solvats sowie deren Gemischte.

2. Vereinigung nach Anspruch 1, dadurch gekenntzeichnet, dass das Hypnotikum mit kurzer Wirkungsdauer Mittel in einer galenischen Formylierung vorliegt, die für eine sofortige oder länger andauernde Freisetzung ausgelegt ist, und das Hypnotikum mit langer Wirkungsdauer in einer galenische Formylierung vorliegt, die für eine sofortige Freisetzung ausgelegt ist.

3. Pharmazeutische Zusammensetzung, **dadurch gekennzeichnet, dass** sie als Werkstoff mindestens ein Hypnotikum mit kurzer Wirkungsdauer, das ausgewählt wird aus Zolpidem, Zopiclon, Eszopiclon, Zaleplon, Melatonin, Ramelteon, Triazolam, Etizolam, Brotizolam, Indiplon, in Form der Base oder eines Additionssalzes, Hydrats oder Solvats sowie deren Gemische, und mindestens ein Hypnotikum mit langer Wirkungsdauer, das ausgewählt wird aus Eplivanserin, in Form der Base oder eines Additionssalzes, Hydrats oder Solvats sowie deren Gemische, und mindestens einen pharmazeutisch annehmbaren Excipienten umfasst.

4. Zusammensetzung nach Anspruch 3, dadurch gekenntzeichnet, dass das Hypnotikum mit kurzer Wirkungsdauer Zolpidem oder eines seiner Salze ist, und dadurch, dass das Hypnotikum mit langer Wirkungsdauer Eplivanserin oder eines seiner Salze ist.

5. Zusammensetzung nach einem der Ansprüche 3 oder 4, dadurch gekenntzeichnet, dass das Hypnotikum mit kurzer Wirkungsdauer und das Hypnotikum mit langer Wirkungsdauer sofort freigesetzt werden.

6. Zusammensetzung nach einem der Ansprüche 3 bis 5, dadurch gekenntzeichnet, dass das Hypnotikum mit kurzer Wirkungsdauer länger andauernd freigesetzt wird und dass das Hypnotikum mit langer Wirkungsdauer sofort freigesetzt wird

7. Zusammensetzung nach einem der Ansprüche 3 bis 6, **dadurch gekennzeichnet, dass** sie aus einer Kapsel besteht, die eine oder mehrere Tabletten mit sofortiger Freisetzung umfasst, die das Hypnotikum mit kurzer Wirkungsdauer enthalten, und eine oder mehrere Tabletten mit sofortiger Freisetzung, die das Hypnotikum mit langer Wirkungsdauer entfalten.

8. Zusammensetzung nach einem der Ansprüche 3 bis 6, dadurch gekenntzeichnet, dass sie aus einer Kapsel besteht, die eine oder mehrere Tabletten mit länger andauernder Freisetzung umfasst, die das Hypnotikum mit kurzer Wirkungsdauer entfalten, und eine oder mehrere Tabletten mit sofortiger Freisetzung, die das Hypnotikum mit langer Wirkungsdauer enthalten.

9. Zusammensetzung nach einen der Ansprüche 3 bis 6, **dadurch gekennzeichnet, dass** sie aus einer Kapsel besteht, die ein Gemisch von Pellets mit sofortiger Freisetzung des Hypnotikums mit kurzer Wirkungsdauer und Pellets mit sofortiger Freisetzung des Hypnotikums mit langer Wirkungsdauer enthält.

10. Zusammensetzung nach einem der Ansprüche 3 bis 6, **dadurch gekennzeichnet, dass** sie aus einer Kapsel besteht, die ein Gemisch von Pellets mit länger andauernder Freisetzung des Hypnotikums mit kurzer Wirkungsdauer und Pellets mit sofortiger Freisetzung des Hypnotikums mit langer Wirkungsdauer enthält.

11. Zusammensetzung nach einem der Ansprüche 3 bis 6, dadurch gekenntzeichnet, dass sie aus einer Tablette besteht, die Pellets mit sofortiger Freisetzung des Hypnotikums mit kurzer Wirkungsdauer und des Hypnotikums mit langer Wirkungsdauer enthält.

12. Zusammensetzung nach einem der Ansprüche 3 bis 6, **dadurch gekennzeichnet, dass** sie aus einer Tablette besteht, die Pellets mit länger andauernder Freisetzung des Hypnotikums mit kurzer Wirkungsdauer und Pellets mit sofortiger Freisetzung des Hypnotikums mit langer Wirkungsdauer enthält.

13. Zusammensetzung nach einen der Ansprüche 3 bis 6, **dadurch gekennzeichnet, dass** sie aus einer magensaftresistent beschichteten Tablette mit länger andauernder Freisetzung besteht, die Pellets mit sofortiger Freisetzung des Hypnotikums mit langer Wirkungsdauer und Pellets mit sofortiger Freisetzung des Hypnotikums mit kurzer Wirkungsdauer umfasst.

14. Zusammensetzung nach einem der Ansprüche 3 bis 6, dadurch gekenntzeichnet, dass sie aus einer Mehrschichttablette besteht, umfassend:
(a) eine oder mehrere Schicht(en) mit sofortiger Freisetzung, die jeweils eine Dosis des Hypnotikums mit langer Wirkungsdauer und gegebenenfalls eine Dosis des Hypnotikums mit kurzer Wirkungsdauer umfassen,
(b) eine oder mehrere Schichten mit länger andauernder Freisetzung, die jeweils eine Dosis des Hypnotikums mit kurzer Wirkungsdauer enthalten, und gegebenenfalls
(c) seine inaktive Schicht, die kein Hypnotikum enthält.

15. Zusammensetzung nach einem der Ansprüche 3 bis 6, **dadurch gekennzeichnet, dass** sie aus eimer trocken überzogenen Tablette besteht, die **dadurch gekennzeichnet ist, dass** sie einen innerem Kern mit länger andauernder Freisetzung umfasst, der das Hypnotikum mit kurzer Wirkungsdauer enthält, und dadurch, dass Überzugsschicht mit sofortiger freisetzung das Hypnotikum mit langer Wirkungsdauer enthält.

16. Verwendung von mindestens einem Hypnotikum mit kurzer Wirkungsdauer, ausgewählt aus Zolpidem, Zopiclon, Eszopicln, Zaleplon, Melatonin, Ramelteon, Triazolam, Etizolam, Brotizolam, Indiplon, in Form der Base oder eines Additionssalzes, Hydrats oder Solvats sowie ihrer Gemische, in Kombination mit mindestens einem Hypnotikum mit langer Wirkungsdauer, ausgewählt aus Eplivanserin, in Form der Base oder eines Additionssalzes, Hydrats oder Solvats sowie ihrer Gemischte, zur Verstellung eines Arzneimittels zur Vorbeugung und/oder Behandlung von Schlafstörungen, insbesondere Dyssomnien, Hypersomnien, Parasomnien, Schlafapnoe, Insomnie, primärer Insomnie, Durchschlafstörungen, Schlaflosigkeit in Zusammenhang mit einer Geisteskrankheit, durch eine Droge, wie Koffein, Alkohol, Amphetamine, Opioide, Anxiolytika induzierter Schlaflosigkeit.
